# EUROPÄISCHE PATENTSCHRIFT

(11) **EP 1 216 650 B1**
(45) Veröffentlichungstag und Bekanntmachung des Hinweises auf die Patenterteilung: **13.04.2005**
(21) Anmeldenummer: 02005797.2
(22) Anmeldetag: 27.09.1996
(51) Int. Cl.: A61B 3/107

(54) **Vorrichtung für die topographische Vermessung einer Oberfläche**
Surface topography measuring apparatus
Dispositif pour la mesure de la topographie d'une surface

(30) Priorität: 17.10.1995 DE 19538567
(43) Veröffentlichungstag der Anmeldung: 26.06.2002
(62) Teilanmeldung aus: 96933432.5
(73) Patentinhaber: Oculus Optikgeräte GmbH, 35582 Wetzlar-Dutenhofen (DE)
(72) Erfinder: Köst, Gert, Diplom-Biologe, 30171 Hannover (DE)
(74) Vertreter: Böck, Bernhard, Dipl.-Ing.

(56) Entgegenhaltungen:
- EP-A- 0 397 962
- EP-A- 0 589 857
- DE-A- 2 641 004
- DE-A- 4 325 494
- GB-A- 2 246 874
- US-A- 5 349 398

## Beschreibung

Die Erfindung betrifft eine Vorrichtung für die topographische Vermessung einer Oberfläche, insbesondere der Hornhaut des menschlichen Auges, mit einem Projektor, bei dem eine von mindestens einer internen Lichtquelle beleuchtete Rasterblende oder dergleichen ein Bildraster auf der Oberfläche abbildet, mit einer Sensorik zur Erfassung des von der Oberfläche reflektierten Bildrasters und mit einer Auswerte-Recheneinheit für die von der Sensorik erfaßten Bilder.

Als Bildraster werden in der Regel Systeme konzentrischer, alternierend heller und dunkler Ringe verwendet. Sie können in verschiedener Weise erzeugt werden. So kann man beispielsweise im Innern eines hohlkugel- oder paraboloidförmigen Gehäuses spiegelnde Ringmarken anbringen, an denen das Licht aus einer in der optischen Achse befindlichen Lichtquelle reflektiert wird, wie das beispielsweise in der Patentschrift DE 32 33 178 C2 vorbeschrieben ist. Eine solche Vorrichtung erfordert einen erheblichen apparativen Aufwand: die Ringmarken müssen außerordentlich präzise ausgebildet und in dem Gehäuse angeordnet sein, um ein regelmäßiges Bildraster zu erzeugen. Bereits kleine Lagefehler der reflektierenden Flächen an den Ringmarken führen zu erheblichen Verzerrungen des Bildrasters. Die Lichtquelle muß ziemlich exakt im Brennpunkt des von den Ringmarken gebildeten Hohlspiegels angebracht sein, wenn das Bildraster gleichförmig ausgeleuchtet sein soll.

Eine stattdessen in einem hohlkegelförmigen Gehäuse befindliche Anordnung eines solchen Ringmarken-Systems ist in der Patentanmeldung EP 0 589 857 A1 beschrieben. Das Gehäuse ist durchweg transparent und wird rückseitig der Ringmarken von einer ringförmigen Lichtquelle ausgeleuchtet. Die Lichtquelle umgibt das Gehäuse koaxial auf dessen Stirnseite mit dem kleinen Durchmesser, in deren Nähe auch noch ein Fixationspunkt in dem Strahlengang abgebildet werden kann, der mittels einer Leuchtdiode erzeugt wird und bei der Ausmessung einer Augen-Hornhaut eine (reproduzierbare) Ruhigstellung des Auges möglich macht. Die Lichtquelle erfordert dabei eine erhebliche Energiezufuhr, ohne daß dadurch eine gleichmäßige Ausleuchtung des Bildrasters sichergestellt wäre. Auch bei dieser Anordnung wirken sich kleine Form- und/oder Lagefehler der Ringmarken nachteilig auf die Gleichförmigkeit des Bildrasters aus.

Aus der Offenlegungsschrift DE 43 25 494 A1 ist es bekannt, den Bildraster mit Hilfe einer hohlkegelstumpfförmigen Rasterblende auszubilden, bei der ringförmige Blendenelemente mit Ringspalten abwechseln. Die Rasterblende wird von einer umgebenden Lichtquelle beleuchtet und erzeugt ebenfalls auf der in ihrer Achse und vor der größeren Stirnseite befindlichen, auszumessenden sphärischen Oberfläche ein Bildraster aus hellen und dunklen Ringen. Eine solche Rasterblende ist relativ leicht exakt herzustellen, so daß das Bildraster ohne viel Aufwand mit guter Regelmäßigkeit erzeugt wird.

Ein auf diese Weise beispielsweise auf einer Augen-Hornhaut abgebildetes Bildraster kann mit Hilfe einer Sensorik ausgemessen werden; dazu ist beispielsweise eine Videokamera geeignet.

Die mittels einer geeigneten Auswerte-Rechnereinheit ausgewerteten, von der Sensorik bereitgestellten und gegebenenfalls mit einem Muster verglichenen Meßergebnisse dienen beispielsweise zur Anpassung von Kontaktlinsen oder zur Führung operativer Instrumente bei der chirurgischen Korrektur von Krümmungsradien, wenn mit der Vorrichtung eine Augen-Hornhaut vermessen wurde.

Als Lichtquellen dienen zumeist zu einem Kreisring geformte Leuchtstofflampen, die viel Raum beanspruchen und im Betrieb große Wärmemengen emittieren. Ihre Blendwirkung ist unerwünscht, wenn eine Augen-Hornhaut ausgemessen werden soll.

Die Erfindung hat sich die Aufgabe gestellt, die geschilderten Mängel abzustellen und eine Vorrichtung der eingangs näher bezeichneten Art so auszubilden, daß eine leistungsfähige, blendfreie Lichtquelle auf engstem Raum installiert werden kann, bei der nur wenig Abwärme anfällt und die deshalb keine konstruktiven Maßnahmen zu deren Ableitung aus dem Bereich der Vorrichtung erfordert.

Erfindungsgemäß wird die Aufgabe dadurch gelöst, daß die interne Lichtquelle aus einer Vielzahl von vorzugsweise gleichmäßig verteilten Leuchtdioden, zusammengesetzt ist. Deren Leuchtdichte kann verhältnismäßig sein, so daß auch die Blendwirkung und die Wärmeentwicklung niedrig gehalten werden können. Sie haben darüberhinaus den Vorteil einer hohen Lebensdauer, so daß die Vorrichtung stets betriebsbereit ist und nur selten mit einer neuen Lichtquelle bestückt werden muß. Erfindungsgemäβ werden für die interne Lichtquelle sogar Leuchtdioden verwendet, welche infrarotes Licht emittieren, so daß bei der Ausmessung einer Augen-Hornhaut Lichtreize im Auge eines Patienten vermieden werden.

Die Rasterblende wird vorteilhaft als Hohlkugelsegment oder als Hohlkegelstumpf ausgebildet, in denen die Blendenelemente am besten als geschlossene Ringe ausgeführt sind, so daß auf diese Weise ein Bildraster mit der gewünschten alternierenden Anordnung konzentrischer, helligkeits-verschiedener Ringe bequem erzeugt werden kann. Die Ringe können dabei unterschiedlich geformte Querschnitte aufweisen,

Ein besonders gleichmäßig ausgeleuchtetes Bildraster erhält man bei einer erfindungsgemäßen Vorrichtung, wenn die Rasterblende auf ihrer der auszumessenden Oberfläche abgewandten Seite mit Abstand von einer an einem Reflektor ausgebildeten, reflektierenden Fläche überfangen ist, bei der weiterhin die interne Lichtquelle in einem auf diese Weise gebildeten, von dem Reflektor und der Rasterblende begrenzten Blendenraum vorgesehen ist, und bei der schließlich die Rasterblende aus einem gut lichtdurchlässigen Werkstoff, insbesondere Plexiglas, besteht, an welchem die den Bildraster ausbildenden, lichtundurchlässigen oder weniger lichtdurchlässigen Blendenelemente vorzugsweise in entsprechend ausgenommenen Ringnuten der Rasterblende angebracht sind.

Das aus der Lichtquelle emittierte Licht wird dabei vielfältig und demzufolge in einer Weise reflektiert, daß die Rasterblende gleichförmig beleuchtet wird und ein homogen ausgeleuchtetes Bildraster auf der auszumessenden Oberfläche erzeugt wird, ohne daß dazu besonders lichtstarke Leuchtdioden vorgesehen werden müßten.

Die Rasterblende wird vorteilhaft als Hohlkugelsegment oder als Hohlkegelstumpf ausgebildet, in denen die Blendenelemente am besten als geschlossene Ringe ausgeführt sind, so daß auf diese Weise ein Bildraster mit der gewünschten alternierenden Anordnung konzentrischer, helligkeits-verschiedener Ringe bequem erzeugt werden kann. Die Ringe können dabei unterschiedlich geformte Querschnitte aufweisen, jedoch ist die Anordnung besonders einfach, wenn dafür solche mit flachrechteckigem Querschnitt verwendet werden, so daß ringförmig geschlossene Bänder vorliegen, die in dazu passenden flachrechteckigen, auf der inneren Oberfläche der Rasterblende eingearbeiteten Ringnuten angebracht sind.

Für eine gleichförmige Ausleuchtung ist es zweckmäßig, wenn die reflektierende Fläche des Reflektors im Bereich des Blendenraumes einen stets gleichen Abstand von der Rasterblende hat, das heißt, daß die Flächen der Rasterblende und des Reflektors einander ähnlich sind.

Die Blendenelemente sind bevorzugt auf der dem Blendenraum abgewandten Seite der Rasterblende befestigt, so daß es auch ohne Schwierigkeiten möglich ist, diese Blendenelemente auf der dem Blendenraum zugewandten Seite mit einer reflektierenden Schicht zu unterlegen, die sich demzufolge, wenn die Blendenelemente einschließlich der reflektierenden Schicht in einer gemeinsamen Ringnut der Rasterblende vorgesehen sind, am Grunde der Ringnut befinden. Durch die Reflexion des von der Lichtquelle in dem Blendenraum emittierten Lichtes an der Schicht wird einer Absorption in den Blendenelemente und deren Aufheizung entgegengewirkt.

Es ist zweckentsprechend, wenn die Blendenelemente in Form eines Systems konzentrischer, voneinander beabstandeter Kreisringe ausgebildet sind, so daß auch das Bildraster einer solchen Anordnung folgt; dessen Deformierung an der auszumessenden Oberfläche ergibt im Regelfall Kreise oder Ellipsen, die rechentechnisch leicht verarbeitet werden können.

Eine besonders bevorzugte Ausführung der Erfindung besteht darin, daß sich in der optischen Achse ein Durchlaß in der Rasterblende befindet. Dabei kann zwischen dem Projektor und der Sensorik in dem aus dem Durchlaß austretenden Strahlengang ein Strahlenteiler zur Einspiegelung einer externen Lichtquelle auf die Oberfläche angeordnet sein. Auf diese Weise kann, ganz unabhängig von dem mit der Rasterblende erzeugten Bildraster, die Vorrichtung auch zur Messung der Reaktion einer Pupille auf plötzliche Blendwirkung gemessen werden, vorausgesetzt, die Sensorik kann die durch die Blendung veranlaßte Bewegung der Pupille erfassen und in digitale Meßsignalsequenzen umsetzen und diese können in der Auswerte-Recheneinheit entsprechend verarbeitet werden; entsprechende Rechenprogramme sind aber verfügbar, so daß die Auswertung einer solchen Messung generell möglich ist. Auf diese Weise ist, über die eigentliche Aufgabenstellung der Erfindung hinaus, eine Vorrichtung geschaffen worden, die, wenn sie im Bereich der Augenheilkunde angewendet wird, gleichzeitig die beiden Funktionen der statischen Ausmessung einer Augen-Hornhaut und der Bestimmung einer Pupillen-Reaktion auszuführen in der Lage ist.

Im einzelnen ist es vorteilhaft, wenn die interne Lichtquelle aus die optische Achse konzentrisch-ringförmig umgebenden Leuchtdioden zusammengesetzt ist. Es können mehrere interne Lichtquellen vorgesehen sein, beispielsweise in der Weise, daß die Lichtquellen an dem äußeren, der auszumessenden Oberfläche zugewandten Rand der Rasterblende und/oder an dem inneren, der auszumessenden Oberfläche abgewandten Rand der Rasterblende, insbesondere an deren Durchlaß, vorgesehen sind. Auf diese Weise kann eine homogene Ausleuchtung mit Sicherheit gewährleistet werden.

Die Erfindung wird nachstehend an Hand der Zeichnung an einem Ausführungsbeispiel näher erläutert. Es zeigen
- Fig. 1: eine erste Ausführung einer erfindungsgemäßen Vorrichtung in einem mittigen Längsschnitt,
- Fig. 2: eine Einzelheit A aus Fig. 1, etwas vergrößert,
- Fig. 3: eine Seitenansicht zu Fig. 1 und
- Fig. 4: eine weitere Ausführung der erfindungsgemäßen Vorrichtung,
sämtlich in vereinfachter, schematischer Darstellung.

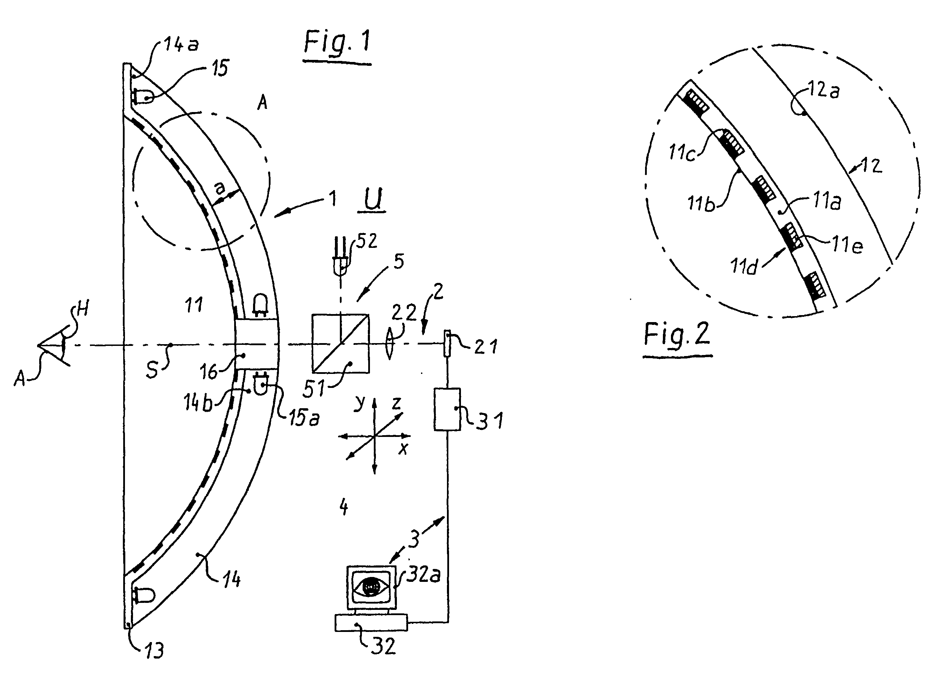

Entsprechend Fig. 1 besteht eine erfindungsgemäße Vorrichtung im wesentlichen aus einem Projektor 1, einer Sensorik 2 und einer Auswerte-Recheneinheit 3; eine Mechanik 4 zur beliebigen Bewegung des Projektors 1 zusammen mit der Sensorik 2 und einer wahlweise angebauten Blendeinrichtung 5 in den drei Raumrichtungen ist durch senkrecht aufeinander stehende Richtungskomponenten x,y,z symbolisch angedeutet.

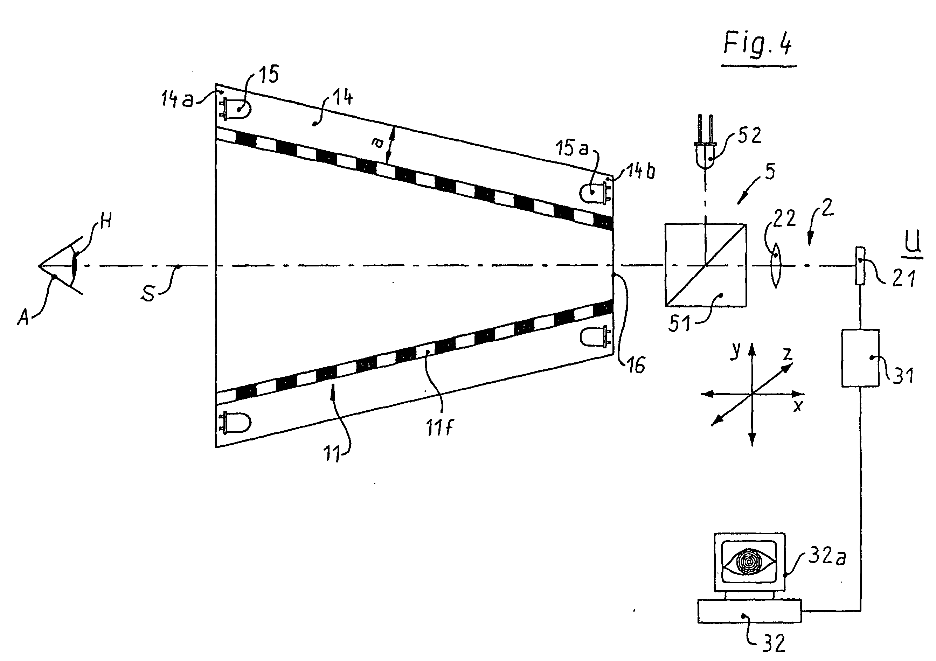

Der Projektor 1 ist in der Fig. 1 als Hohlkugelsegment ausgebildet, in der Fig. 4 hingegen als Hohlkegelstumpf, während aber in beiden Ausführungen ansonsten die übrigen Bauund Funktionselemente übereinstimmend angeordnet sind.

In der Fig. 1 ist zu sehen, daß der Projektor 1 aus einer Rasterblende 11 und einem Reflektor 12 besteht, die in einem gemeinsamen, in der Zeichnung nur schematisch angedeuteten Gehäuse 13 so angebracht sind, daß der Reflektor 12 mit seiner reflektierenden Fläche 12a zu der Oberfläche der Rasterblende 11 stets den gleichen Abstand a aufweist und so ein gekrümmter Blendenraum 14 gebildet wird. Die innere, geschlossene Oberfläche des Reflektors 12 ist stark reflektierend, so daß der Blendenraum 14 gleichmäßig lichterfüllt ist, wenn eine interne Lichtquelle 15 eingeschaltet ist. Deren Wirkung kann durch eine weitere Lichtquelle 15a gesteigert werden. Beide Lichtquellen 15,15a bestehen aus einer Vielzahl ringförmig am Umfang des Projektors 1 in dichter Anordnung verteilter Leuchtdioden.

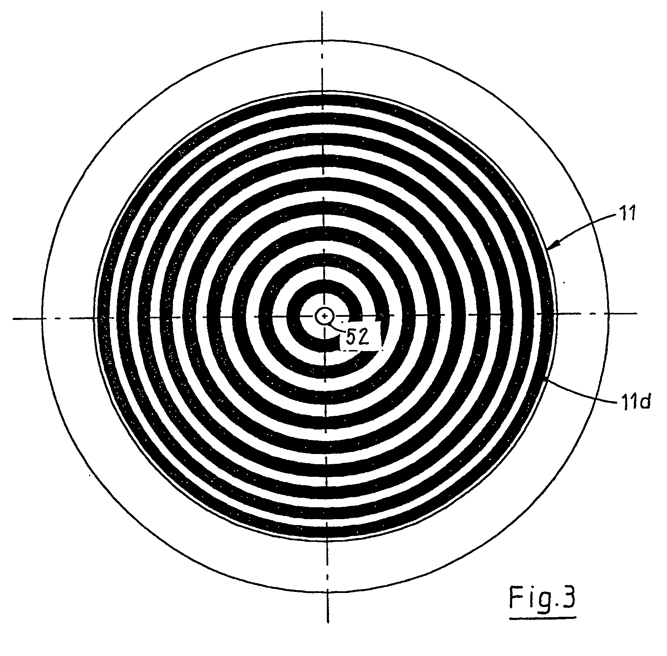

In der optischen Achse des Projektors 1 ist das Auge A eines Patienten angedeutet, an dem die sphärische Form der Hornhaut H mit der Vorrichtung ausgemessen werden soll. Eine Einrichtung zur Raumorientierung des Auges A ist dabei als selbstverständlich in der Zeichnung weggelassen worden; das Auge A kann im übrigen aber frei bewegt werden. Das Auge A erblickt im Betrieb der Vorrichtung den Projektor 1 so, wie es die Fig. 3 zeigt.

In der Symmetrieachse des Projektors 1 ist an diesem ein Durchlaß 16 vorgesehen, durch den der Strahlengang S aus dem Auge A auf die im Hintergrund U des Projektors 1 befindliche Sensorik 2 gerichtet ist; sie besteht in einfacher Weise aus einer Videokamera 21 mit einem vorgeschalteten Objektiv 22.

Die Lichtquelle 15 befindet sich innerhalb des Blendenraumes 14 an dessen äußerem, dem Patienten zugekehrten Rand 14a, während die zusätzliche und nur zeitweise erforderliche und sonst abgeschaltete Lichtquelle 15a den inneren Rand 14b des Blendenraumes 14 um den Durchlaß 16 herum umgibt.

Die Sensorik 2 ist mit einer Auswerte-Recheneinheit 3 verbunden, mit deren Hilfe die von der Videokamera 21 stammenden und dort bereits digitalisierten Aufnahmen des Bildrasters ausgewertet werden; das eingehende, in einem Prozessor 31 vorverarbeitete Aufnahmematerial wird in einem Personalcomputer 32 einerseits über ein Display 32a wiedergegeben, und andererseits wird dort das eingespeiste Bildraster der Berechnung beispielsweise der Krümmungsradien der Hornhaut H des Auges A zugrunde gelegt. Das Bildraster besteht danach aus einem beispielsweise zu Ellipsen gekrümmten Ringsystems des ursprünglich entsprechend Fig. 3 aus konzentrischen (Kreis-) Ringen zusammengesetzten Bildrasters.

In dem Strahlengang S ist zwischen dem Projektor 1 und der Sensorik 2 in der optischen Achse ein Strahlenteiler 51 angeordnet, der, wie in den Fig. 1 und 4 zu erkennen ist, hier aus einem Prismensystem besteht, jedoch auch aus teildurchlässigen ebenen Spiegeln zusammengesetzt sein kann. Über den Strahlenteiler 51 kann eine externe Lichtquelle 52 in dem Auge A abgebildet werden. Auf diese Weise kann man ganz unabhängig von der übrigen Vorrichtung einen Blendreiz auf dem Auge A erzeugen, so daß dessen Reaktion mit Hilfe der ohnehin verfügbaren Sensorik 2 erfaßt und von der Auswerte-Recheneinheit 3 auch numerisch bestimmt werden kann. Ein spezielles Gerät ist für eine solche Messung dementsprechend nicht mehr erforderlich. Der Strahlenteiler 51 bildet zusammen mit der Lichtquelle 52 die Blendeinrichtung 5.

In der Einzelheit der Fig. 2 ist gut zu erkennen, daß die Rasterblende 11 aus mehreren Teilen zusammengesetzt ist. In einem transparenten Korpus 11a sind auf dessen dem Blendenraum 14 abgewandten Oberfläche 11b kreisringförmige Ringnuten 11c eingetieft, in denen die ebenfalls kreisringförmigen Blendenelemente 11d angeordnet und mit einer reflektierenden Schicht 11e unterlegt sind, welche auftreffendes Licht aus einer Lichtquelle 15,15a in den Blendenraum 14 reflektiert.

Wie Fig. 4 zeigt, kann die Rasterblende 11 auch einfach aus alternierend angeordneten Ringschlitzen 11f und Blendenelementen 11d bestehen.

Besonders wirkungsvoll arbeitet die Vorrichtung, wenn für die internen Lichtquellen 15,15a Leuchtdioden Verwendung finden, die - für den Patienten unsichtbares - infrarotes Licht abstrahlen, das aber von einer geeigneten Sensorik 2 erfaßt und auf dem Display 32a sichtbar gemacht werden kann. Das Auge A ist dann sehr ruhig gestellt, und eine ausreichende Blendwirkung mittels der externen Lichtquelle 52 wird bereits bei niedrigem Lichtstrom aus der Lichtquelle 52 erhalten, so daß das Auge A geschont wird.

## Patentansprüche

1. Vorrichtung für die topographische Vermessung einer Oberfläche, insbesondere der Hornhaut (H) des menschlichen Auges (A), mit einem Projektor (1), bei dem eine von mindestens einer internen Lichtquelle (15,15a) beleuchtete Rasterblende (11) oder dergleichen ein Bildraster auf der Oberfläche (H) abbildet, mit einer Sensorik (2) zur Erfassung des von der Oberfläche (H) reflektierten Bildrasters und mit einer Auswerte-Recheneinheit (3) für die von der Sensorik (2) erfaßten Bilder, **dadurch gekennzeichnet, daß** die interne Lichtquelle (15,15a) aus einer Vielzahl von vorzugsweise gleichmäßig verteilten Leuchtdioden zusammengesetzt ist, wobei für die internen Lichtquellen (15,15a) Leuchtdioden verwendet werden, welche infrarotes Licht emittieren, und wobei sich in der optischen Achse ein Durchlaß (16) in der Rasterblende (11) befindet, und wobei zwischen dem Projektor (1) und der Sensorik (2) in dem aus dem Durchlaß (16) austretenden Strahlengang (S) eine Blendeinrichtung zur Erzeugung eines Blendreizes auf dem Auge (A), bestehend aus einer externen Lichtquelle und einem Strahlenteiler (51) zur Einspiegelung der externen Lichtquelle (52) auf die Oberfläche (H) angeordnet ist.

2. Vorrichtung nach Anspruch 1, **dadurch gekennzeichnet, daß**
(a) die Rasterblende (11) auf ihrer der auszumessenden Oberfläche (H) abgewandten Seite mit Abstand (a) von einer an einem Reflektor (12) ausgebildeten, reflektierenden Fläche (12a) überfangen ist,
(b) die interne Lichtquelle (15,15a) in einem auf diese Weise gebildeten, von dem Reflektor (12) und der Rasterblende (11) begrenzten Blendenraum (14) vorgesehen ist und
(c) die Rasterblende (11) aus einem gut lichtdurchlässigen Werkstoff, insbesondere Plexiglas, besteht, an welchem die den Bildraster ausbildenden, lichtundurchlässigen oder weniger lichtdurchlässigen Blendenelemente (11d) vorzugsweise in entsprechend ausgenommenen Ringnuten (11c) der Rasterblende (11) angebracht sind.

3. Vorrichtung nach Anspruch 1 oder 2, dadurch ge**kennzeiahnet, daß** die Rasterblende (11) als Hohlkugelsegment ausgebildet ist.

4. Vorrichtung nach Anspruch 1 oder 2, **dadurch gekennzeichnet , daß** die Rasterblende (11) als Hohlkegelstumpf ausgebildet ist.

5. Vorrichtung nach einem der Ansprüche 1 bis 4, **dadurch gekennzeichnet, daß** die Blendenelemente (11d) als geschlossene Ringe vorzugsweise flachrechteckigen Querschnitts ausgebildet sind.

6. Vorrichtung nach einem der Ansprüche 1 bis 5, **dadurch gekennzeichnet, daß** die reflektierende Fläche (12a) des Reflektors (12) im Bereich des Blendenraumes (14) einen stets gleichen Abstand (a) von der Rasterblende (11) hat.

7. Vorrichtung nach einem der Ansprüche 1 bis 6, **dadurch gekennzeichnet, daß** die Blendenelemente (11d) auf der dem Blendenraum (14) abgewandten Seite der Rasterblende (11) befestigt sind.

8. Vorrichtung nach einem der Ansprüche 1 bis 7, **dadurch gekennzeichnet, daß** die Blendenelemente (11d) auf ihrer dem Blendenraum (14) zugewandten Seite mit einer reflektierenden Schicht (11e) unterlegt sind.

9. Vorrichtung nach einem der Ansprüche 1 bis 8, **dadurch gekennzeichnet, daß** die Blendenelemente (11d) in Form eines Systems konzentrischer, voneinander beabstandeter Kreisringe ausgebildet sind.

10. Vorrichtung nach einem der Ansprüche 1 bis 9, **dadurch gekennzeichnet, daß** die interne Lichtquelle (15,15a) aus die optische Achse konzentrisch-ringförmig umgebenden Leuchtdioden zusammengesetzt ist.

11. Vorrichtung nach einem der Ansprüche 1 bis 10, **dadurch gekennzeichnet, daß** mehrere Lichtquellen (15,15a) vorgesehen sind.

12. Vorrichtung nach Anspruch 11, **dadurch gekennzeichnet, daß** die interne Lichtquelle (15) an dem äußeren, der auszumessenden Oberfläche () zugewandten Rand (14a) der Rasterblende (11) vorgesehen ist.

13. Vorrichtung nach Anspruch 11, **dadurch gekennzeichnet, daß** die interne Lichtquelle (15a) an dem inneren, der auszumessenden Oberfläche (H) abgewandten Rand (14b) der Rasterblende (11), insbesondere an deren Durchlaß (16), vorgesehen ist.

## Claims

1. A device for the topographic measuring of a surface, in particular the cornea (H) of the human eye (A), with a projector (1), in which a scanning device (11) or the like illuminated by at least one internal light source (15,15a) images an image pattern on the surface (H), with a sensor unit (2) for detecting the image pattern reflected from the surface (H) and with a computer system (3) for the images detected by the sensor unit (2), **characterised in that** the internal light source (15,15a) is composed of a plurality of preferably uniformly distributed light-emitting diodes, whereby light-emitting diodes which emit infrared light are used for the internal light sources (15,15a), and whereby a passage (16) is located in the optical axis in the scanning device (11), and whereby arranged between the projector (1) and the sensor unit (2) in the beam path (S) exiting from the passage (16) is a diaphragm device for generating a diaphragm stimulus on the eye (A), comprising an external light source and a beam divider (51) for activating an external light source (52) on the surface (H).

2. The device as claimed in Claim 1, **characterised in that**
(a) the scanning device (11) is encased by a reflecting face (12a) imaged on a reflector (12) on its side averted from the surface (H) to be measured at a distance (a),
(b) the internal light source (15,15a) is provided in a diaphragm chamber (14) formed in this manner, delimited by the reflector (12) and the scanning device (11), and
(c) the scanning device (11) comprises a good light-permeable material, in particular Plexiglas, on which the light-impermeable or less light-permeable diaphragm elements (11d) imaging the image pattern are preferably housed in correspondingly recessed annular grooves (11c) of the scanning device (11).

3. The device as claimed in Claim 1 or 2, **characterised in that** the scanning device (11) is configured as a hollow spherical segment.

4. The device as claimed in Claim 1 or 2, **characterised in that** the scanning device (11) is configured as a hollow truncated cone.

5. The device as claimed in any one of Claims 1 to 4, **characterised in that** the diaphragm elements (11d) are configured as closed rings with a preferably flat rectangular cross-section.

6. The device as claimed in any one of Claims 1 to 5, **characterised in that** the reflecting face (12a) of the reflector (12) has a constantly uniform distance (a) from the scanning device (11) in the region of the diaphragm chamber (14).

7. The device as claimed in any one of Claims 1 to 6, **characterised in that** the diaphragm elements (11d) are attached to the side of the scanning device (11) averted from the diaphragm chamber (14).

8. The device as claimed in any one of Claims 1 to 7, **characterised in that** the diaphragm elements (11d) are underlaid on their side facing the diaphragm chamber (14) with a reflecting layer (11e).

9. The device as claimed in any one of Claims 1 to 8, **characterised in that** the diaphragm elements (11d) are configured in the form of a system of concentric circular rings spaced apart from one another.

10. The device as claimed in any one of Claims 1 to 9, **characterised in that** the internal light source (15,15a) is composed of light-emitting diodes surrounding the optical axis concentrically and annularly.

11. The device as claimed in any one of Claims 1 to 10, **characterised in that** several light sources (15,15a) are provided.

12. The device as claimed in Claim 11, **characterised in that** the internal light source (15) is provided on the outer edge (14a) of the scanning device (11), facing the surface (H) to be measured.

13. The device as claimed in Claim 11, **characterised in that** the internal light source (15a) is provided on the inner edge (14b) of the scanning device (11), averted from the surface (H) to be measured, in particular on its passage (16).

## Revendications

1. Dispositif pour la mesure topographique d'une surface, notamment de la cornée (H) de l'oeil humain (A), avec un projecteur (1), sur lequel une plaque de masquage (11) éclairée par au moins une source lumineuse interne (15, 15a) ou similaire reproduit la trame d'une image sur la surface (H), avec un système de capteurs (2) pour la détection de la trame d'image reflétée par la surface (H) et avec une unité de calcul/d'évaluation (3) pour les images détectées par le système de capteurs (2), **caractérisé en ce que** la source lumineuse (15, 15a) est composée d'une pluralité de diodes lumineuses réparties de préférence de façon homogène,
des diodes lumineuses émettant de la lumière infrarouge étant utilisées pour la source lumineuse interne (15, 15a) et
un passage (16) se trouvant dans l'axe optique, dans la plaque de masquage (11), et
un dispositif d'éblouissement pour la génération d'une stimulation par éblouissement de l'oeil (A), constitué d'une source lumineuse externe et d'un séparateur de faisceaux (51) étant disposé entre le projecteur (1) et le système de capteurs (2), dans la trajectoire du faisceau (S) émanant du passage (16), pour la réflexion de la source lumineuse externe (52) sur la surface (H).

2. Dispositif selon la revendication 1, **caractérisé en ce que**
(a) sur sa face qui est opposée à la surface (H) devant être mesurée, la plaque de masquage (11) est doublée d'une surface réfléchissante (12a) formée sur un réflecteur (12),
(b) la source lumineuse interne (15, 15a) est prévue dans un espace de diaphragme (14) formé de cette façon et délimité par le réflecteur (12) et par la plaque de masquage (11) et
(c) la plaque de masquage (11) est constituée en un matériau bien transparent, notamment en plexiglas, sur lequel les éléments de diaphragme (11d) opaques ou moins transparent formant la trame d'image sont montés de préférence dans des rainures annulaires (11c) creusées en conséquence dans la plaque de masquage (11).

3. Dispositif selon la revendication 1 ou 2, **caractérisé en ce que** la plaque de masquage (11) est conçue sous la forme d'un segment à sphère creuse.

4. Dispositif selon la revendication 1 ou 2, **caractérisé en ce que** la plaque de masquage (11) est conçue sous la forme d'un cône creux tronqué.

5. Dispositif selon l'une quelconque des revendications 1 à 4, **caractérisé en ce que** les éléments du diaphragme (11d) sont conçus sous la forme de bagues fermées, avec une section transversale de préférence de forme rectangulaire plate.

6. Dispositif selon l'une quelconque des revendications 1 à 5, **caractérisé en ce que** dans la zone de l'espace de diaphragme (14), la surface réfléchissante (12a) du réflecteur (12) présente une distance (a) toujours égale par rapport à la plaque de masquage (11).

7. Dispositif selon l'une quelconque des revendications 1 à 6, **caractérisé en ce que** les éléments de diaphragme (11d) sont fixés sur la face de la plaque de masquage (11) qui est opposée à l'espace de diaphragme (14).

8. Dispositif selon l'une quelconque des revendications 1 à 7, **caractérisé en ce que**, sur leur face dirigée vers l'espace de diaphragme (14), les éléments de diaphragme (11d) sont placés sur une couche réfléchissante (11e).

9. Dispositif selon l'une quelconque des revendications 1 à 8, **caractérisé en ce que** les éléments de diaphragme (11d) sont conçus sous la forme d'un système de bagues toriques concentriques, distancées les unes par rapport aux autres.

10. Dispositif selon l'une quelconque des revendications 1 à 9, **caractérisé en ce que** la source lumineuse interne (15, 15a) est composée de diodes lumineuses entourant de façon concentrique/annulaire l'axe optique.

11. Dispositif selon l'une quelconque des revendications 1 à 10, **caractérisé en ce que** plusieurs sources lumineuses (15, 15a) sont prévues.

12. Dispositif selon la revendication 11, **caractérisé en ce que** la source lumineuse interne (15) est prévue sur le bord externe (14a) de la plaque de masquage (11), qui est dirigé vers la surface à mesurer (H).

13. Dispositif selon la revendication 11, **caractérisé en ce que** la source lumineuse interne (15a) est prévue sur le bord interne (15a) de la plaque de masquage (11) qui est opposé à la surface à mesurer (H), notamment sur son passage (16).
